# EUROPEAN PATENT APPLICATION

(11) **EP 1 918 280 A1**
(43) Date of publication of application: **07.05.2008**
(21) Application number: 06123547.9
(22) Date of filing: 06.11.2006
(51) Int. Cl.: C07D 211/26, C07C 41/01

(54) **Process for the preparation of 2,5-bis-(2,2,2-trifluoroethoxy)-N-(2-piperidylmethyl)-benzamide and salts thereof**

(71) Applicant: "Joint Stock Company Grindeks", Riga 1057 (LV)
(72) Inventor: Zicane, Daina, LV-1058, Riga (LV); Jaunbergs, Janis, Grindeks, joint stock company, LV-1013, Riga (LV)

(57) **Abstract**

A process for the preparation of Flecainide acetate, its salts, in particular its pharmaceutically acceptable salts, and intermediates thereof is described wherein 2,5-dibromotoluene is used as a starting material. The method involves a technique for preparing the starting material 2,5-*bis*(2,2,2-trifluroethoxy)toluene in high yields by reacting 2,5-dibromotoluene with 2,2,2-trifluoroethanol in the presence of a base and a copper-containing catalyst.

## Description

### Field of the invention

The present invention relates to an improved process for the preparation of 2,5-*bis*(2,2,2-trifluoroethoxy)-*N*-(2-piperidylmethyl)benzamide (international non-proprietary name - "Flecainide") and salts, in particularly pharmaceutically acceptable salts, thereof.

### Background art

Flecainide base, pharmaceutically acceptable salts thereof, and in particular its hydrochloride form, are generally described in US-A-3900481.

Flecainide is an active ingredient used in human therapy as an anti-arrhythmic agent, as described in US-A-4005209.

There are many processes described in the art for the synthesis of this compound, for example:

US-B-4024175 describes the preparation of Flecainide starting from 1,4-dibromobenzene which is transformed by reaction with trifluoroethoxy compounds and acetylating agents into 2,5-*bis*(2,2,2-trifluoroethoxy)acetophenone, which after subsequent gives Flecainide.

One process for preparing Flecainide disclosed in GB-A-2045760 starts from 2,5-*bis* (2,2,2-trifluoroethoxy)benzoic acid. 2,5*-Bis* (2,2,2-trifluoroethoxy)benzoic acid is prepared by a multistage process, comprising the conversion of 1,4-dibromobenzene into 1,4-*bis*(2,2,2-trifluoroethoxy)benzene by reacting 8 equivalents of 2,2,2-trifluoroethanol per mol of dibromobenzene, and then acetylating the 1,4-*bis*(2,2,2-trifluoroethoxy) benzene to form 2,5-*bis*(2,2,2-trifluoroethoxy) acetophenone. The 2,5-*bis*(2,2,2-trifluoroethoxy)acetophenone then is oxidized to the corresponding 2,5-*bis*(2,2,2-trifluoroethoxy)benzoic acid.

The 2,5-*bis*(2,2,2-trifluoroethoxy)benzoic acid then is converted into its acid chloride and reacted with 2-(aminomethyl)piperidine to form Flecainide in one step. Alternatively, the acid chloride can be reacted with 2-(aminomethyl)pyridine, followed by catalytic hydrogenation of the pyridine ring, to form Flecainide.

A disadvantage of using 1,4-dibrombenzene to form 1,4-*bis*(2,2,2-trifluoroethoxy)benzene is that the process requires the reaction of 8 equivalents of 2,2,2-trifluoroethanol while only 2 equivalents are theoretically needed. The use of less than 8 equivalents of 2,2,2-trifluoroethanol, however, results in an incomplete conversion into 1,4-*bis* (2,2,2-trifluoroethoxy)benzene with the starting material and 1-bromo-4-(2,2,2-trifluoroethoxy)benzene as the main impurities.

The one step-process has a further disadvantage in that the acid chloride reacts non-selectively with both nitrogen atoms of the 2-(aminomethyl)piperidine resulting in a mixture of the two acylated isomers. This is the main reason why the two-step process via the pyridine intermediate presently is commercially preferred. A further disadvantage is due to the fact the acid chloride intermediate is a liquid which cannot be stored for a long period of time but must be used immediately after its preparation.

The multi step-process for obtaining 2,5-*bis*(2,2,2-trifluoroethoxy)benzoic acid can also be mentioned as a further disadvantage.

2,5-*Bis*(2,2,2-trifluoroethoxy)benzoic acid can also be prepared from 1-bromo-4-fluorobenzene (see WO-A-02066413) or from 2-bromo-5-chlorobenzoic acid (see WO-A-9902498).

For the preparation of 2,5-*bis*(2,2,2-trifluoroethoxy)benzoic acid from 2-bromo-5-chlorobenzoic acid sodium hydride in combination with *N,N-*dimethylformamide is used. However, it is known that using sodium hydride in combination with *N,N*-dimethylformamide can lead to explosion (see BUCKLEY, J.. "Report on thermal reaction", published in "Chem. Eng. News". 1982, vol.60, no.28, p.5., POND, DAVID, "Sodium hydride and DMF", published in "Chem. Eng. News". 1982, vol. 60, no. 37, p. 5,43. This is the main disadvantage of the process disclosed in WO-A-9902498 Furthermore, the use of 1-bromo-4-fluorobenzene as a starting material is comparatively expensive.

The method disclosed in WO-A-02066413 is a multi-step process that includes obtaining alkyl 2,5-*bis*(2,2,2-trifluoroethoxy)phenylglyoxalate as an intermediate product which is oxidized to form 2,5-*bis*(2,2,2-trifluoroethoxy)benzoic acid. However, this approach has only a limited commercial utility due to the high cost.

The process disclosed in WO-A-9902498 starts from the cyanomethyl ester of 2,5-*bis*(2,2,2-trifluoroethoxy)benzoic acid which selectively reacts with 2-(aminomethyl)piperidine to furnish Flecainide.

The process disclosed in WO-A-02066413 starts from 2,5-*bis*(2,2,2-trifluoroethoxy)benzene which is converted into 2,5-*bis*(2,2,2-trifluoroethoxy)benzoyl chloride, and then is reacted with 2,2,2-trifluoroethanol in the presence of an appropriate base to produce 2,5-*bis*(2,2,2-trifluoroethoxy)benzoate. Finally, the 2,5-*bis*(2,2,2-trifluoroethoxy)benzoate is reacted with 2-(aminomethyl)pyridine to form 2.5-*bis*(2,2,2-trifluoroethoxy)pyridylbenzamide which then is reduced to form Flecainide.

The prior art process for the manufacture of 2,5-*bis*(2,2,2-trifluorethoxy)-*N-*(pyrid-2-yl-methyl)benzamide has several disadvantages which have been overcome by the present invention namely:
1. the introduction of the trifluorethoxy group substituents at the aromatic ring was previously achieved
   - by employing costly sulfonate leaving groups, such as trifluoromethane sulfonate or perfluorobutane sulfonate or
   - by treating 1,4-dibromobenzene or 5-bromo-2-chlorobenzoic acid with 2,2,2-trifluorethanol in dimethylformamide in the presence of NaH;
2. the derivatization of the trifluorethoxy substituted aromatic system to produce 2,5-*bis*(trifluorethoxy)benzoic acid or its derivatives was conducted by Friedel-Crafts acylation, followed by chlorination; and
3. the 2,5-*bis*(2,2,2-trifluorethoxy)benzoic acid conversion into 2,5*-bis* (2,2,2-trifluorethoxy)-*N*-(pyrid-2-yl-methyl)benzamide previously required the preparation of acyl chloride or trichloro acetophenone, the handling of these intermediates representing unnecessary challenges.

### Disclosure of the invention

### Technical problem

The objective underlying the present invention is to overcome the above disadvantages of the prior art. In particular it is the objective of the present invention to provide a novel commercial process for the preparation of Flecainide acetate salt or their salts, in particular their pharmaceutically acceptable salts, starting from commercially available dihalotoluenes of formula I, where X¹ and X² each are F, Cl, Br or I.

### Technical solution

The above objective is achieved according to the present invention by a sequential combination of four process steps, starting from 2,5-dibromotoluene:
1. replacement of the 2,5-dibromotoluene aromatic bromine substituents by trifluoroethoxy groups, and, thus, preparation of 2,5-*bis*(trifluoroethoxy)toluene or any of the intermediate substitution products 2-bromo-5-trifluorethoxytoluene and 5-bromo-2-trifluorethoxytoluene,
2. oxidation of 2,5-*bis*(trifluorethoxy)toluene to produce 2,5-*bis*(trifluorethoxy)benzoic acid,
3. activation of 2,5-*bis*(trifluorethoxy)benzoic acid in-situ with a chloroformate, and substitution of the leaving carbonate group with 2-(aminomethyl)pyridine, and
4. hydrogenation of 2,5-*bis*(2,2,2-trifluorethoxy)-*N*-(pyrid-2-yl-methyl)benzamide to obtain Flecainide acetate.

### Advantageous effects

The following Reaction Scheme 1 outlines the present method of preparation of Flecainide acetate. The method includes the following advantages:
1. use of more environmentally friendly materials and solvents, respectively, which have low toxicity;
2. use of metallic sodium instead sodium hydride in combination with *N,N* - dimethylformamide as safer method in industrial production;
3. production of the desired final product in higher yields;
4. amenability for large scale production which does not require specialized equipment.

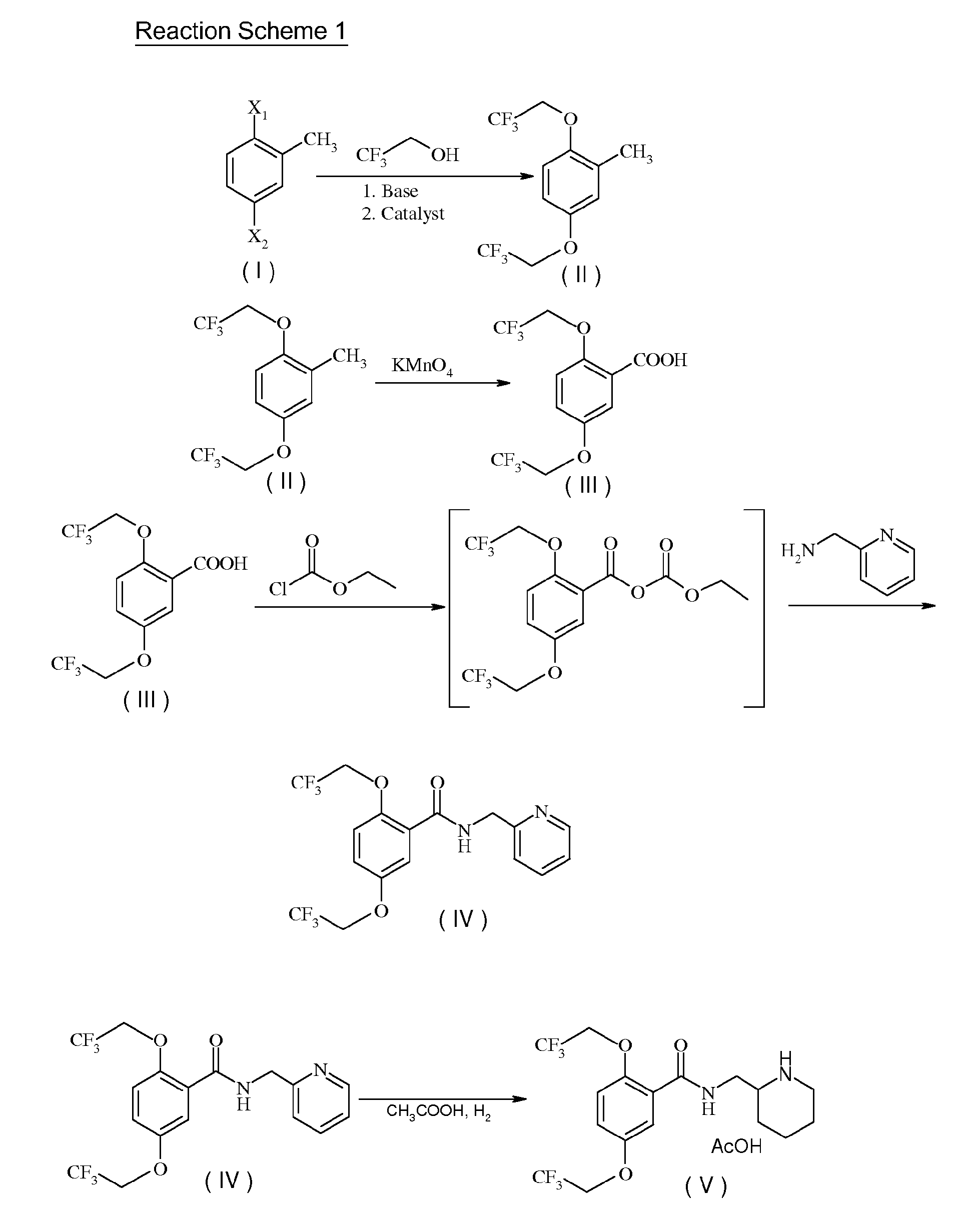

Thus, the subject matter of the present invention is a process for preparing a compound of formula VI: 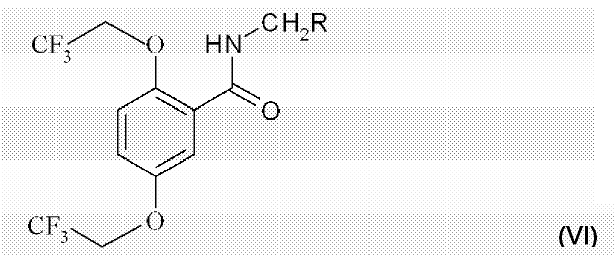 wherein R is a 2-piperidyl or 2-pyridyl radical, and salts, in particular pharmaceutically acceptable salts, thereof, the process comprising the following steps:

a) preparation of a compound of formula II 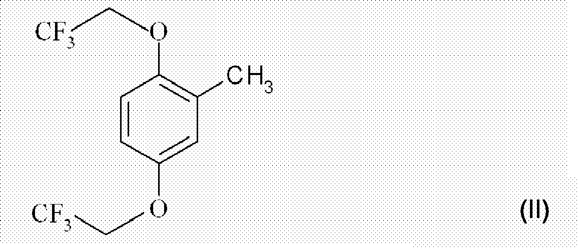 from a compound of formula I 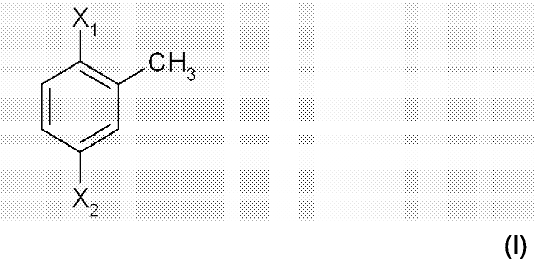 by reaction of the 2,5-dihalotoluene of formula I with a strong base and 2,2,2-trifluoroethanol in the presence of a catalyst in an aprotic solvent to form 2,5-*bis*(2,2,2-trifluoroethoxy)toluene of formula II,

b) oxidation of compound II to yield 2,5-*bis*(2,2,2-trifluorethoxy)benzoic acid of formula III;

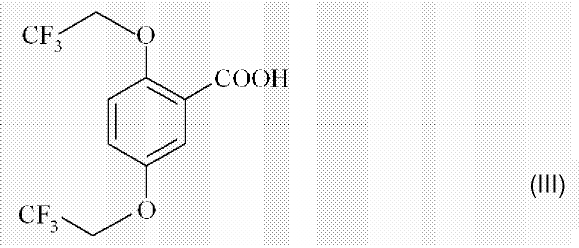

c) activatiing the benzoic acid of formula III with a carboxylic acid ester and thereafter reacting the mixed anhydride of benzoic acid of formula III with an amine of the formula RCH₂NH₂, where R is 2-pyridyl; and

d) converting the compound of formula VI into a salt, in particular into a pharmaceutically acceptable salt, thereof.

Subject-matter of the present invention is also a process for the preparation of the key intermediate 2,5-*bis*(2,2,2-trifluoroethoxy)benzoic acid beginning with a starting material of formula I, where X¹ and X² each are F,Cl,Br and I.

Reaction of starting material of formula I with 2,2,2-trifluoroethanol in the presence of a base and a suitable catalyst such as a copper catalyst provides compounds of formula II in high yield (e.g., 92 %).

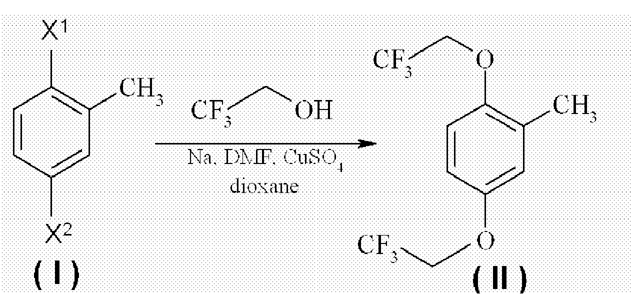

Compound of formula II is oxidized with permanganate compounds like sodium permanganate or potassium permanganate to yield a compound of formula III, which is the main intermediate product for preparation of Flecainide acetate, compound of formula V.

Compound of formula III is activated with a carboxylic acid ester and after reaction with the primary amino group of 2-(aminomethyl)pyridine produces compound of formula IV.

It is yet another subject-matter of present invention to prepare and use the intermediate 2,5-dibromotoluene for the manufacture of Flecainide acetate, compound of formula V.

Heterogeneous catalytic hydrogenation of compound IV in the presence palladium and platinum on carbon in a suitable solvent, e.g., water, is carried out in a solution of an aliphatic carboxylic acid (e.g. glacial acetic acid) for obtaining Flecainide acetate, compound of formula V.

### Best mode for carrying out the invention

As indicated above, the method of the present invention involves the initial preparation of 2,5-*bis*(2,2,2-trifluoroethoxy)toluene (II) by reacting 2,5-dihalotoluene (I) with 2,2,2-trifluoroethanol in the presence of a strong base and a copper containing catalyst. The 2,2,2-trifluoroethanol reacts in about 2-10-fold molar excess of the 2,5-dihalotoluene to replace the 2,5-dihalotoluene aromatic halogen substituents with trifluoroethoxy groups.

Bases that enable the reaction include alkali metals, e.g., metallic sodium. A copper containing material is used as a catalyst in the reaction, e.g., copper (II) sulphate. For performing reaction *N,N*-dimethylformamide can be used as an aprotic solvent. The best mode for carrying out the reaction is a temperature of about 85 to 105°C.

In accordance with a practicularly preferred embodiment, the 2,5-dibromotoluene (I), where X¹ and X² is Br, is reacted with sodium 2,2,2-trifluoro-ethoxide (produced by reacting 2,2,2-trifluoroethanol with Na) in *N,N-*dimethylformamide in the presence of CuSO₄ at about 85 to 105°C.

The 2,5-*bis*(2,2,2-trifluoroethoxy)toluene (II) is thereafter oxidized in the presence of a suitable oxidant to produce 2,5-*bis*(2,2,2-trifluorethoxy)benzoic acid (III). As oxidants potassium permanganate and sodium permanganate can be used.

2,5-*Bis*(2,2,2-trifluorethoxy)benzoic acid (III) is activated with ethyl chloroformate and thereafter is reacted with the primary amino group of 2-(aminomethyl)pyridine to yield 2,5-*bis*(2,2,2-trifluorethoxy)-*N*-(pyrid-2-yl-methyl)benzamide (IV).

2,5-*bis*(2,2,2-trifluorethoxy)-*N*-(pyrid-2-yl-methyl)benzamine (IV) is hydrogenated in the presence of a platinum or palladium on carbon catalyst in a suitable solvent, e.g., water. The reduction can be performed in a hydrogen atmosphere at 4-6 atmospheres. The reaction may be carried out within about 3 hours. The residual filtrate is reacted with a suitable base, e.g. NaOH, to produce the Flecainide free base. The Flecainide free base then is converted into Fecainide acetate (V). The free base is thus reacted with glacial acetic acid to form the acetate. The reaction may be preformed at 31 °C.

The present invention will be described in more detail by referring to the following non-limiting examples.

**Example 1**

**Preparation of 2,5-*bis*(2,2,2-trifluoroethoxy)toluene (II)**

2,2,2-trifluoroethanol (55.0 g, 0.550 mol) was added to dioxane (125 mL) in a glass vessel fitted with a reflux condenser. Sodium metal (11.5 g, 0.500 mol) was added in portions of 2-3 grams to the solution, resulting in a temperature increase from 22°C to 90°C. The solution was stirred at 85-105°C until the sodium dissolution was completed, then *N,N-*dimethylformamide (100 mL) was added, followed by 2,5-dibromotoluene (I) (42.5 g, 0.170 mol) and anhydrous copper (II) sulphate (2.9 g, 0.018 mol). The reaction mixture was stirred at 95-100°C for 4 hours, and then cooled to 25-30°C and poured into 900 ml of a cold (5-10°C) 40% aqueous methanol solution.

Concentrated hydrochloric acid was added (~25 mL, 0.300 mol), until pH=1-2. The crystallization suspension was stirred for 1 hour at -5 to 0°C, the solid white precipitate was filtered, after which the reaction vessel and the product cake on the filter were rinsed with 50 mL of water. The intermediate 2,5-*bis*(2,2,2-trifluoroethoxy)toluene (II) was dried at ambient temperature and pressure for 5 hours, then at 22 to 24°C and at a reduced pressure for 4 hours. The 2,5-*bis*(2,2,2-trifluoroethoxy)toluene was obtained as a white or off-white powder (45.5 g, 92%) having a melting point of 37°C to 42°C.

**Example 2**

**Preparation of 2,5-*bis*(2,2,2-trifluorethoxy)benzoic acid (III)**

The 2,5-*bis*(2,2,2-trifluoroethoxy)toluene (II) (220 g, 0.986 mol) was added to magnetically stirred pyridine (1550 mL) at 18-23°C. The reaction mixture was stirred under a reflux. Thereafter a 10 % aqueous NaOH solution (20 mL, 0.050 mol) and copper (II) sulphate (0.27 g, 0.0017 mol) were added to the reaction mixture.

The reaction mixture was heated at 81 °C for 1 hour at which point a 40% aqueous sodium permanganate solution (2290 g, 6.45 mol) was added in portions within a time period of 6.5 hours. After the addition of an aqueous sodium permanganate solution the reaction mixture was stirred at 83 to 93°C for 75 minutes. A precipitate of manganese oxides was separated by filtration at 80-95°C, the manganese oxides was rinsed on the filter with 5 liters of hot (80°C) water. The combined filtrates were cooled to 9°C in 50 minutes.

The crystallization suspension was filtered to obtain damp cake of unreacted 2,5-*bis*(2,2,2-trifluoroethoxy)toluene (II), which was removed thereafter.

The filtrate of reaction mixture was mixed with activated charcoal (20.0 g) and stirred for 75 minutes; the precipitate of charcoal was separated by filtration and liquid phase was acidified to pH=1-2 using concentrated HCl. The crystallization solution was filtrated and the product cake on the filter was rinsed with 200 mL of water.

The intermediate 2,5-*bis*(2,2,2-trifluorethoxy)benzoic acid (III) was dried at reduced pressure for 18 hours. The 2,5-*bis*(2,2,2-trifluorethoxy)benzoic acid was obtained as an off-white powder (210.2 g, 81.6%) having a melting point of 121°C to 125°C.

**Example 3**

Pyridine (310 mL), water (175 mL) and 2,5-*bis*(2,2,2-trifluoroethoxy)toluene (44.4 g, 0.154 mol) were mixed in a 1 liter round bottom flask, the pH was adjusted to 11-12 with sodium hydroxide.

The mixture was heated to 85°C and treated with potassium permanganate in 6 portions of 34 grams each, for a total of 204 g (1.29 mol). The permanganate addition rate was such as to maintain the temperature at 90-100°C. The reaction mixture was maintained at 85-95°C for an additional hour and filtered while hot. The manganese oxide precipitate was washed with water and the washings were combined with the pyridine-water filtrate, then cooled to 5-10°C and filtered to remove the unreacted starting material.

The product was isolated by acidifying the filtrate with concentrated hydrochloric acid to pH=1-2 and removing the white precipitate by filtration. After washing on the filter with water and drying under reduced pressure 2,5-*bis*(2,2,2-trifluoroethoxy)benzoic acid was obtained as white powder in 37.2 g (0.116 mol, 75.2%) yield having a melting point of 121°C to 125°C.

**Example 4**

**Preparation of 2,5-*bis*(2,2,2-trifluorethoxy)-*N*-(pyrid-2-yl-methyl)benzamide (IV)**

2,5-*bis*(2,2,2-trifluoroethoxy)benzoic acid (III) (100 g, 0.314 mol), triethylamine (50.2 mL) and ethyl acetate (630 mL) were mixed in a 4-liter 3 neck round bottom flask, fitted with a thermometer, a dropping funnel and a calcium chloride drying tube. The reaction mixture was stirred and cooled to 5 to 10°C. A solution of ethyl chloroformate (33.0 mL, 0.345 mol) in ethyl acetate (315 mL) was added dropwise to the reaction mixture. Thereafter the reaction mixture was stirred at 5 to 10°C for 30 minutes. A solution of 2-(aminomethyl)pyridine (36.0 mL, 0.350 mol) in ethyl acetate (315 mL) was added dropwise to the mixture. The reaction mixture was stirred at 15-25°C for 1 hour. Water (600 mL) was added, and the aqueous layer was separated and extracted with ethyl acetate (300 mL). The organic layer was concentrated *in vacuo* to two thirds of the original volume and hexanes (750 mL) were added. The resulting suspension was stirred at 5-10°C for 1 hour.

The precipitate was separated by filtration and washed with hexanes (250 mL). The yield was 116.4g (91.7%) of 2,5-*bis*(2,2,2-trifluorethoxy)-*N-*(pyrid-2-yl-methyl)benzamide (IV) having a melting point 101°C -106°C.

**Example 5**

**Preparation of 2,5-*bis*(2,2,2-trifluoroethoxy)-*N*-(2-piperidylmethyl)benzamide acetate (V)**

2,5-*bis*(2,2,2-trifluoroethoxy)-*N*-(pyrid-2-yl-methyl)benzamide (1205 g, 2.88 mol), glacial acetic acid (4000 mL), 5 percent platinum on carbon with 61 % water content (105.5 g) and 10 percent palladium on carbon with 60% water content (28.7 g) were mixed in a 10 liter autoclave.

The autoclave with the reaction mixture was heated to 70°C while rapid stirring over 20-30 minutes, under a hydrogen pressure of 0.39×10⁶ Pa. Pressure was increased to 0.59×10⁶ Pa. 5 minutes later. Hydrogen uptake stopped within 3 hours at 69-72°C, but hydrogenation was continued for additional 2 hours. The reaction mixture was filtered to remove the catalyst.

Approximately 60% of the solvent was removed by distillation at reduced pressure. Water (35 L) was added to the distillation residue, the mixture was then treated with an aqueous NaOH solution to pH=13-14. The solid white precipitate was filtered, dried at reduced pressure and dissolved in ethyl acetate (12 L). The solution was heated to 73°C for 5 minutes, filtered and to the filtrate was added glacial acetic acid (175 ml) at 31 °C.

The precipitates were separated by filtration and then rinsed with ethyl acetate (500 ml). The damp cake was dried at reduced pressure for 14 hours at 20°C to give Flecainide acetate as a white solid in 1173 g (86 %) yield having a melting point of 146 to 152°C.

## Claims

1. Process for the preparation of Flecainide, as Flecainide base, and salts, in particular pharmaceutically acceptable salts thereof, comprising the synthesis on the intermediate 2,5-*bis*(2,2,2-trifluoroethoxy)toluene (II), defined in step a), by the reaction of 2,5-dihalotoluene (I) with 2,2,2-trifluoroethanol 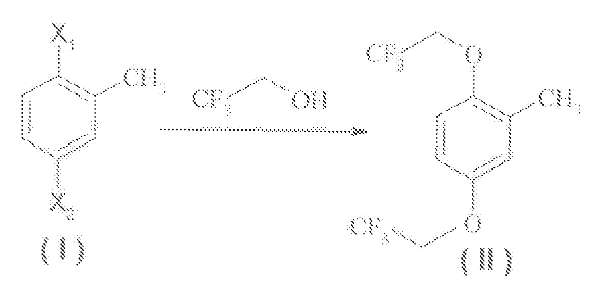 in the presence of a strong inorganic base and copper containing catalyst in an aprotic solvent.

2. The process according to claim 1, wherein in the reaction step a) X₁ and X₂ are selected from halogen, preferably Br, I, Cl and F, wherein X₁ and X₂ more preferably are Br.

3. The process according to claim 2, wherein the X₁ and X₂ are Br, i.e. the compound of formula (I) is 2,5-dibromotoluene.

4. The process according to claim 1, wherein the molar ratio between 2,5-dihalotoluene and 2,2,2-trifluoroethanol is comprised from 1:2 to 1:10.

5. The process according to claim 4, wherein the molar ratio is 1:6.

6. The process according to claim 1, wherein the reaction in step a) is preferably carried out in the presence of a strong inorganic base selected from the group of alkali metals.

7. The process according to claim 6, wherein the strong inorganic base is metallic sodium.

8. The process according to claim 1, wherein in the reaction in step a) copper containing catalyst is copper (11) sulphate.

9. The process according to claim 1, wherein the reaction in step a) is carried out in an aprotic solvent mixture.

10. The process according to claim 9, the aprotic solvent mixture contains *N,N*-dimethylformamide.

11. The process according to claim 1, wherein the teacher in step a) is carried out at a temperature of from 30°C to 140°C; preferably from 70°C to 120°C; and more preferably from 85°C to 105°C.

12. The process according to claim 1, wherein step a) leads to formation of the intermediate 2,5-*bis*(2,2,2-trifluoroethoxy)toluene, and the oxidation reaction of the 2,5-*bis*(2,2,2-trifluoroethoxy)toluene, defined in step b), leads to the intermediate 2,5-*bis*(2,2,2-trifluoroethoxy)benzoic acid.

13. The process according to claim 12, wherein the oxidation reaction in step b) is carried out with a suitable oxidant.

14. The process according to claim 13, wherein the suitable oxidant is potassium permanganate or sodium permanganate.

15. The process according to claim 12, wherein step b) formation of the intermediate 2,5-*bis*(2,2,2-trifluoroethoxy)benzoic acid, and the reaction of the intermediate 2,5-*bis*(2,2,2-trifluoroethoxy)benzoic acid with 2-aminomethylpyridine, defined in step c), leads to 2,5-*bis*(2,2,2-trifluoroethoxy)-*N-*(pyrid-2-yl-methyl)benzamide.

16. The process according to claim 15, wherein the reagent for carboxylic group activation is selected from the group consisting of carbonic acid chlorides.

17. The process according to claim 16, wherein the reagent is ethyl chloroformate.

18. The process according to claim 15, wherein the molar ratio between the intermediate 2,5-*bis*(2,2,2-trifluoroethoxy)benzoic acid and 2-(aminomethyl)pyridine is comprised from 1:0.8 to 1:3.

19. The process according to claim 18, wherein the molar ratio is 1:1.

20. The process according to claim 15, wherein step c) leads to formation of the intermediate 2,5-*bi*s(2,2,2-trifluoroethoxy)-*N*-(pyrid-2-methyl)benzamide, and the heterogeneous catalytic hydrogenation of the intermediate 2,5-*bis*(2,2,2-trifluoroethoxy)-*N*-(pyrid-2-yl-methyl)benzamide by using suitable catalysts, defined in step d), leads to Flecainide base.

21. The process according to claim 20, wherein suitable catalysts are palladium and platinum on carbon in a suitable solvent, preferably water.

22. The process according to claim 20, wherein hydrogenation is carried out in a suitable solution of an aliphatic carboxylic acid.

23. The process according to claim 22, wherein the aliphatic carboxylic acid is glacial acetic acid.

24. The process according to claim 20 wherein the reaction in step a) is carried out at a temperature of from 30°C to 140°C; preferably from 50°C to 80°C; and more preferably from 65°C to 75°C.

25. The process according to claim 20, wherein steps a), b), c) and d) as described above lead to Flecainide monoacetate.

26. The process according to claim 25, wherein the Flecainide monoacetate is purified by crystallisation in ethyl acetate.
